(19) Europäisches Patentamt
European Patent Office
Office européen des brevets

(11) Veröffentlichungsnummer: 0 104 583
B1

(12) # EUROPÄISCHE PATENTSCHRIFT

(45) Veröffentlichungstag der Patentschrift: **06.09.89**

(51) Int. Cl.⁴: **C 07 D 493/08** // (C07D493/08, 307:00, 307:00)

(21) Anmeldenummer: **83109297.8**

(22) Anmeldetag: **20.09.83**

(54) **Verfahren zur Herstellung von 7-oxabicyclo(2,2,1)Hept-5-en-Derivaten.**

(30) Priorität: **24.09.82 DE 3235399**

(43) Veröffentlichungstag der Anmeldung: **04.04.84 Patentblatt 84/14**

(45) Bekanntmachung des Hinweises auf die Patenterteilung: **06.09.89 Patentblatt 89/36**

(84) Benannte Vertragsstaaten: **AT BE CH DE FR GB IT LI LU NL SE**

(56) Entgegenhaltungen:
**CHEMICAL ABSTRACTS, Band 80, 1974, Seite 412, Nr. 120667w, Columbus, Ohio, US; A.W. McCULLOCH u.a.: "Influence of Lewis acids on the Diels-Alder reaction. V. Reaction of furan with dimethyl acetylenedicarboxylate" & CAN. J. CHEM. 1973, 51(24), 4125-36**
**TETRAHEDRON LETTERS, Band 23, Nr. 50, 1982, Seiten 5299-5302, Pergamon Press Ltd., London, GB; F. BRION: "On the Lewis acid catalyzed Diels-Alder reaction of furan. Regio- and stereospecific synthesis of substituted cyclohexenols and cyclohexadienols"**

(73) Patentinhaber: **Chemische Fabrik Stockhausen GmbH, Bäkerpfad 25, D-4150 Krefeld (DE)**

(72) Erfinder: **Landscheidt, Alfons, Dr., Dipl.-Chem., Kölner Strasse 390, D-4150 Krefeld (DE)**

(74) Vertreter: **Klöpsch, Gerald, Dr.-Ing., An Gross St. Martin 6, D-5000 Köln 1 (DE)**

EP 0 104 583 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 7-Oxabicyclo(2,2,1)hept-5-en-Derivaten.

Die Umsetzung von Estern α,β-ungesättigter Säuren mit Furanen nach der Diels-Alder-Reaktion zu 7-Oxabicyclo(2,2,1)hept-5-en-Derivaten gemäß Formelschema 1 ist bekannt:

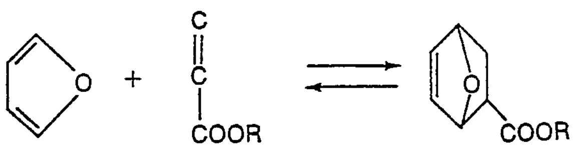

Die Reaktion führt wie alle Diels-Alder-Reaktionen bis zu einem Gleichgewichtsumsatz, der von den eingesetzten Reaktionskomponenten sowie von den thermodynamischen Reaktionsparametern Temperatur und Druck abhängig ist.

Nachteilig ist bisher die lange Reaktionszeit, die auf eine sehr geringe Reaktionsgeschwindigkeit zurückzuführen ist. So benötigt die Reaktion Furan/Ethylacrylat mehrere Wochen, bis der Umsatz 21% beträgt (M. P. Kunstmann et al., Journ. Am. Chem. Soc. 82 (1962), 4119). Die Umsetzung von Furan mit Methylacrylat führt unter analogen Bedingungen nur zu einem Umsatz von 18% (R. J. Quellette et al., Journ. Org. Chem. Soc. 33 (1968), 4303). Eine Reduzierung der Reaktionszeit auf einige Stunden kann durch eine technisch aufwendige Druckerhöhung auf 15 kbar erreicht werden (W. G. Dauben, H. O. Krabbenhoft, Journ. Am. Chem. Soc. 98 (1976), 1992). Die Reaktionszeit kann auf einige Tage verkürzt werden, wenn statt des Esters ein reaktiveres Dienophil wie Acryloylchlorid eingesetzt wird. Das Diels-Alder-Addukt des Furans entsteht dabei in hoher Ausbeute. Wird dagegen 2-Methylfuran als Dien eingesetzt, bildet sich das entsprechende Produkt der substituierenden Addition gemäß Formelschema 2 (T. A. Eggelte et al., Heterocycles 4 (1976), 19–22):

2) 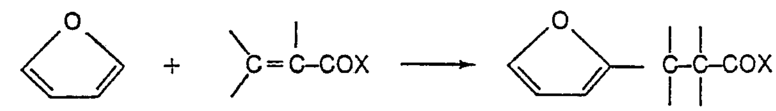

In «Chemical Abstracts, Band 80, 1974, Seite 412, Nummer 120 667w» wird die Umsetzung von Furan mit Acetylendicarbonsäuredimethylester in Gegenwart von Aluminiumtrichlorid beschrieben. Nach einstündiger Reaktionsdauer haben sich 20% Monoaddukt und 22% Diaddukt gebildet. Außerdem tritt als Nebenreaktion eine beträchtliche Dienpolymerisation auf, wodurch die nicht zum Mono- oder Diaddukt umgesetzten Ausgangskomponenten verloren gehen.

Aufgabe der Erfindung ist daher die Schaffung eines Verfahrens zur Herstellung von Diels-Alder-Addukten aus Furanen und Acrylsäureestern in möglichst hoher Ausbeute und kurzer Reaktionsdauer.

Überraschenderweise wurde jetzt gefunden, daß man die Umsetzung von Acrylsäureestern mit Furanen zu 7-Oxabicyclo(2,2,1)hept-5-en-2-carbonsäureestern in einigen Minuten durchführen kann, wenn man die Reaktion in Gegenwart von katalytischen Mengen Lewis-Säure vornimmt. Dies ist umso erstaunlicher, als Lewis-Säuren bekanntlich die Addition-Substitution-Reaktion begünstigen (A. P. Dunlop, F. N. Peters, ‹The Furans› Reinold Publish. Co., New York, 1953).

Die Aufgabe wird dadurch gelöst, daß man Acrylsäureester von Alkoholen mit 1 bis 22 C-Atomen mit gegebenenfalls substituierten Furanen in Gegenwart katalytischer Mengen einer Lewis-Säure umsetzt.

Die Umsetzung kann bei Raumtemperatur oder darunter durchgeführt werden. Tiefe Temperaturen verschieben das Gleichgewicht zur Produktseite. Üblicherweise wird die Umsetzung in einem Temperaturbereich zwischen 0 und 40 °C, vorzugsweise zwischen 15 und 25 °C durchgeführt.

Als Katalysatoren können alle bekannten Lewis-Säuren eingesetzt werden. Genannt seien $AlCl_3$, $TiCl_4$, $SnCl_4$, $FeCl_3$ und $BF_3 \cdot OEt_2$. Besonders bevorzugt wird $AlCl_3$ eingesetzt. Als Esterkomponente wird Acrylsäuremethylester, als Furankomponente Furan bevorzugt. Zur Durchführung der Umsetzung wird die Lewis-Säure bevorzugt in dem vorgelegten α,β-ungesättigten Ester gelöst und das Furan unter Kühlung zugetropft. Das Diels-Alder-Addukt bildet sich spontan. Wenn die exotherme Reaktion abgeklungen ist, wird nach Zersetzung der Lewis-Säure mit Wasser die organische Phase abgetrennt und zuerst nicht reagiertes Furan und α,β-ungesättigter Ester abdestilliert. Das so erhaltene Rohprodukt kann bei größeren Ansätzen nur unter schonenden Bedingungen bevorzugt kontinuierlich – z. B. im Dünnschichtverdampfer und bevorzugt im Vakuum – destilliert werden, da sonst die Zersetzung in die Ausgangskomponenten, die Retro-Diels-Alder-Reaktion, eintritt.

Die Reaktionsprodukte sind wertvolle Zwischenprodukte für viele Synthesen, z. B. die Herstellung hochreiner N-substituierter Acrylsäureamide, durch Umsetzung mit primären oder sekundären Aminen und thermische Spaltung der Produkte.

Die folgenden Beispiele erläutern die Erfindung:

Beispiel 1:

Zu 100 g (1 mol) Ethylacrylat werden 4,5 g (0,033 mol) $AlCl_3$ zugegeben und unter Rühren gelöst. Danach werden unter Kühlung bei 20 bis 25 °C 68 g (1 mol) Furan zugetropft. Nach 1 Stunde ist die exotherme Reaktion beendet, man rührt 1 Stunde bei 25 °C nach und versetzt dann mit 30 g

Wasser. Die organische Phase wird abgetrennt. Zuerst wird das nicht umgesetzte Furan und Ethylacrylat bei einem Druck von 10 Torr, dann der Rückstand bei einem Druck von 0,1 Torr destilliert. Das 2-Carbomethoxy-7-oxabicyclo(2,2,1)hept-5-en siedet bei 60 °C und wird in einer Ausbeute von 67,2 g (40% d. Th.) erhalten.

IR, NMR und CH-Analyse bestätigen die Struktur.

Beispiel 2:

43 g (0,5 mol) Methylacrylat werden vorgelegt und 2,3 g (0,016 mol) $AlCl_3$ zugegeben und unter Rühren gelöst. Bei 20 bis 25 °C wird jetzt unter Kühlung 48 g (0,5 mol) 2,5-Dimethylfuran zugetropft. Man hält die Temperatur bei 20 °C, bis die schwach exotherme Reaktion beendet ist und rührt noch 1 Stunde nach. Dann versetzt man mit 20 g Wasser, trennt die organische Phase ab und destilliert das nicht umgesetzte 2,5-Dimethylfuran und das Methylacrylat bei einem Druck von 10 Torr ab. Der Rückstand wird bei einem Druck von 0,1 Torr destilliert. Das 2-Carbomethoxy-1,4-dimethyl-7-oxabicyclo(2,2,1)hept-5-en siedet bei 40 °C und wird in einer Ausbeute von 27,3 g (30% d. Th.) erhalten.

IR, NMR und CH-Analyse bestätigen die Struktur.

Beispiel 3:

1290 g (15 mol) Methacrylat werden vorgelegt und 67,5 g (0,5 mol) $AlCl_3$ zugegeben und unter Rühren gelöst. Bei 20 bis 25 °C wird jetzt unter Kühlung 1050 g (15,4 mol) Furan zugetropft. Man hält die Temperatur bei 20 °C, bis die exotherme Reaktion beendet ist und rührt noch 1 Stunde bei 20 °C und dann 3 Stunden bei 10 °C nach. Dann versetzt man mit 300 g Wasser, trennt die organische Phase ab und destilliert das nicht umgesetzte Furan und das Methylacrylat bei einem Druck von 10 Torr und einer Badtemperatur von 60 °C am Rotationsverdampfer ab. Anschliessend wird das Vakuum auf 1 Torr erniedrigt und weitere flüchtige Bestandteile abgezogen. Das Rohprodukt wird bei einem Vakuum von 1 Torr und einer Thermostat-Temperatur von 95 °C in einen Dünnschichtverdampfer eingespeist. Es stellt sich eine Siedetemperatur von ca. 70 °C ein. Das 2-Carbomethoxy-7-oxabicyclo(2,2,1)hept-5-en wird in einer Ausbeute von 1060 g (45,9% d. Th.) erhalten.

IR, NMR und CH-Analyse bestätigen die Struktur.

Beispiel 4:

Zu 64 g (0,5 mol) Butylacrylat werden 3,3 g (0,013 mol) $SnCl_4$ zugegeben und unter Rühren gelöst. Danach werden unter Kühlung bei 20 bis 25 °C 34 g (0,5 mol) Furan zugetropft. Nach 1 Stunde ist die exotherme Reaktion beendet, man rührt 1 Stunde bei 25 °C nach und versetzt mit 30 g Wasser. Die organische Phase wird abgetrennt. Zuerst wird das nicht umgesetzte Furan und Butylacrylat bei einem Druck von 10 Torr, dann der Rückstand bei einem Druck von 0,02 Torr destilliert. Das 2-Carbobutoxy-7-oxabicyclo(2,2,1)hept-5-en siedet bei 65 °C und wird in einer Ausbeute von 34,3 g (35% d. Th.) erhalten.

IR, NMR und CH-Analyse bestätigen die Struktur.

Beispiel 5:

Zu 120 g (0,5 mol) Dodecylacrylat werden 2,5 g (0,013 mol) $TiCl_4$ zugegeben und unter Rühren gelöst. Danach werden unter Kühlung bei 20 bis 25 °C 34 g (0,5 mol) Furan zugetropft. Nach 1 Stunde ist die exotherme Reaktion beendet, man rührt 1 Stunde bei 25 °C nach und versetzt mit 30 g Wasser. Die organische Phase wird abgetrennt. Das nicht umgesetzte Furan und Dodecylacrylat wird bei einem Druck von 1 Torr abdestilliert. Das 2-Carbododecyloxy-7-oxabicyclo(2,2,1)hept-5-en wird in einer Ausbeute von 69,3 g (45% d. Th.) erhalten.

IR, NMR und CH-Analyse bestätigen die Struktur.

**Patentansprüche**

1. Verfahren zur Herstellung von 7-Oxabicyclo-(2,2,1)hept-5-en-2-carbonsäureestern aus Furanen und Acrylsäureestern, dadurch gekennzeichnet, daß Ester der Acrylsäure mit Alkoholen von 1 bis 22 C-Atomen mit gegebenenfalls substituierten Furanen in Gegenwart katalytischer Mengen einer Lewis-Säure umgesetzt werden.

2. Verfahren nach Anspruch 1, dadurch gekennzeichnet, daß die Umsetzung in einem Temperaturbereich zwischen 0 und 40 °C, vorzugsweise zwischen 15 und 25 °C durchgeführt wird.

3. Verfahren nach einem der Ansprüche 1 bis 2, dadurch gekennzeichnet, daß als Lewis-Säure $AlCl_3$, $TiCl_4$, $SnCl_4$, $FeCl_3$ oder $BF_3 \cdot OET_2$ eingesetzt wird.

4. Verfahren nach einem der Ansprüche 1 bis 3, dadurch gekennzeichnet, daß als Esterkomponente Acrylsäuremethylester eingesetzt wird.

5. Verfahren nach einem der Ansprüche 1 bis 4, dadurch gekennzeichnet, daß als Furankomponente Furan eingesetzt wird.

6. Verfahren nach einem der Ansprüche 1 bis 5, dadurch gekennzeichnet, daß nach Zersetzung der Lewis-Säure und Abtrennung nicht-umgesetzter Ausgangskomponenten das erhaltene Rohprodukt unter schonenden Bedingungen, bevorzugt im Vakuum, destilliert wird.

**Claims**

1. A process for the production of 7-oxabicyclo(2.2.1)-hept-5-ene-2-carboxylic acid esters from furanes and acrylic acid esters, characterized in that esters of the acrylic acid are reacted with alcohols of 1 to 22 carbon atoms with optionally substituted furanes in the presence of catalytic quantities of a Lewis acid.

2. A process according to claim 1, characterized in that the reaction is carried out at temperatures between 0 and 40 °C, preferably between 15 and 25 °C.

3. A process according to claims 1 to 2, characterized in that $AlCl_3$, $TiCl_4$, $SnCl_4$, $FeCl_3$, or $BF_3{\cdot}OET_2$ are used as Lewis acid.

4. A process according to claims 1 to 3, characterized in that acrylic acid methyl ester is used as ester component.

5. A process according to claims 1 to 4, characterized in that furane is used as furane component.

6. A process according to claims 1 to 5, characterized in that after decomposition of the Lewis acid and separation of non-reacted starting components, the crude product obtained is distilled under mild conditions, preferably under vacuum.

**Revendications**

1. Procédé de préparation d'esters de l'acide 7-oxabicyclo(2,2,1)-hept-5-ène-2-carboxylique à partir de furannes et d'esters de l'acide acrylique, caractérisé en ce que l'on fait réagir les esters de l'acide acrylique avec des alcools qui comportent de 1 à 22 atomes de carbone sur des furannes éventuellment substitués, en présence de proportions catalytiques d'un acide de Lewis.

2. Procédé suivant la revendication 1, caractérisé en ce que l'on réalise la réaction dans une plage de températures qui fluctue de 0 à 40 °C, plus particulièrement de 15 à 25 °C.

3. Procédé suivant l'une des revendications 1 et 2, caractérisé en ce que l'on utilise $AlCl_3$, $TiCl_4$, $SnCl_4$, $FeCl_3$ ou $BF_3{\cdot}OET_2$, à titre d'acide de Lewis.

4. Procédé suivant l'une des revendications 1 à 3, caractérisé en ce que l'on utilise l'acrylate de méthyle à titre de composant du genre ester.

5. Procédé suivant l'une des revendications 1 à 4, caractérisé en ce que l'on utilise le furanne à titre de composant du genre furanne.

6. Procédé suivant l'une des revendications 1 à 5, caractérisé en ce qu'après la décomposition de l'acide de Lewis et la séparation des composants de départ non entrés en réaction, on distille le produit brut obtenu dans des conditions ménagées, de préférence, sous vide.